# FASCICULE DE BREVET EUROPEEN

(11) **EP 1 841 389 B1**
(45) Date de publication et mention de la délivrance du brevet: **07.03.2012**
(21) Numéro de dépôt: 06709158.7
(22) Date de dépôt: 24.01.2006
(51) Int. Cl.: A61J 3/07, A61K 9/00, A61F 6/14

(54) **Procédé de fabrication d'un réservoir contenant une substance active diffusant à travers le reservoir et installation pour sa mise en oeuvre**
Verfahren zur Herstellung eines Reservoirs mit einer durch das Reservoir diffundierten aktiven Substanz und dessen Installation
Method for making a reservoir containing an active substance diffused through the reservoir and installation therefor

(30) Priorité: 25.01.2005 FR 0500758
(43) Date de publication de la demande: 10.10.2007
(73) Titulaire: Bayer Pharma Aktiengesellschaft, 13353 Berlin (DE)
(72) Inventeur: LAFONT, Charles-Dominique, F-03300 Cusset (FR)
(74) Mandataire: Suominen, Kaisa Liisa
(86) Numéro de dépôt international: PCT/FR2006/000159
(87) Numéro de publication internationale: WO 2006/079709

(56) Documents cités:
- EP-A- 0 652 738
- EP-A- 1 400 258
- US-A- 4 920 727
- US-A- 5 230 207
- US-A- 5 400 804

## Description

L'invention a trait à un procédé de fabrication d'un réservoir contenant une substance active et adapté pour être introduit dans une cavité naturelle d'un être vivant, ce réservoir étant enveloppé par une membrane perméable à la substance active.

De tels réservoirs sont utilisés comme dispositifs contraceptifs intra-utérins également dénommés stérilets hormonaux. Ce type de réservoir comprend une membrane tubulaire, généralement réalisée dans un matériau à base de silicone. Un produit contenant notamment une matrice, à base de silicone, et une hormone contraceptive est enveloppé par cette membrane. La membrane est perméable à l'hormone, celle-ci diffusant, de manière régulière, de l'intérieur du réservoir vers la cavité utérine. De tels stérilets comprennent également un support inerte, à base de polymère, sur lequel est fixé le réservoir afin d'assurer le maintien en position du dispositif dans la cavité utérine.

Ce mode d'administration de produits contraceptifs permet d'utiliser de très faibles doses journalières d'hormones progestatives, celle-ci étant délivrées directement au niveau de l'organe cible. L'efficacité de ces dispositifs est comparable à celle des contraceptifs à base d'hormone à prise par voie orale. Leur durée d'action se situe entre deux et cinq ans et, à la différence des stérilets comprenant, en lieu et place du réservoir, un fil de cuivre, ils sont non allergiques. Ces stérilets hormonaux sont notamment utilisés en cas d'hyperménorrhée, ce qui se produit fréquemment avec des stérilets à base de cuivre.

La membrane entourant le réservoir est formée à partir d'un tube de silicone de faible diamètre et d'une épaisseur de paroi la plus petite possible. Les contraintes techniques ne permettent pas, néanmoins, de réaliser des tubes de silicone d'une épaisseur de paroi inférieure à 0,4 millimètre et d'un diamètre inférieur à 1,5 milimètres. Or, la diffusion de l'hormone à travers la membrane doit être lente, continue et régulière, quelle que soit la quantité d'hormones présentes dans le réservoir. Ceci n'est possible, en application des lois de FICK sur la diffusion membranaire, que pour des épaisseurs de membrane beaucoup plus faibles.

Compte tenu des dimensions maximales du réservoir imposées par la morphologie, il est connu, par exemple de EP-A-0 652 738, d'insérer un cylindre d'un produit contenant une hormone dans un tube de silicone. Cette insertion permet d'étendre radialement le tube de silicone et d'amener, par étirement, la paroi du tube à l'épaisseur voulue en jouant sur les propriétés élastiques du silicone. Dans ce procédé un tube de silicone est maintenu en place et reçoit, dans sa lumière, une ébauche. Cette ébauche comprend un moyen d'injection d'air sous pression qui dilate le tube. On remplace ensuite, progressivement, cette ébauche par un cylindre, réalisé au préalable, de produit contenant hormone. L'étape finale permet de solidariser la membrane autour du cylindre en diminuant la pression de l'air à l'intérieur du tube.

Le document US 4,920,727 décrit une installation pour réaliser un réservoir contenant une substance active. Le réservoir est enveloppé par une membrane preméable à la substance active. L'installation comprend un organe de maintien d'un tube destiné à former ladite membrane, un organe d'injection d'un produit contenant la substance active ainsi qu'un organe de fermeture d'au moins une extrémité du tube.

Dans le même esprit, US-A-5 400 804 décrit un procédé de réalisation d'un réservoir tubulaire contenant uns substance contraceptive. Un réservoir tubulaire, entouré d'une gaine, coiffe une aiguille disposée dans un moule. L'aiguille envoie de l'air pour augmenter le diamètre interne du réservoir. Une fois le diamètre augmenté, une tige formant support remplace l'aiguille.

De tels procédés nécessitent de disposer d'une longueur de tube identique à la longueur du cylindre de produit. Autrement dit, celui-ci doit être fabriqué au préalable et découpé à la bonne longueur, il en est de même pour le tube de silicone. Par ailleurs, l'injection d'air sous pression, peut être mal maîtrisée et/ou le tube mal maintenu lors de cette injection. Il peut alors se former, sur la membrane du tube, une hernie dans laquelle se loge une bulle d'air. Enfin, de tels systèmes ne permettent pas de solidariser efficacement la membrane externe avec le cylindre interne.

C'est à ces inconvénients qu'entend plus particulièrement remédier l'invention en proposant un procédé et un équipement pour réaliser un réservoir contenant une substance active dans lequel une membrane mince est aisément mise en place, tout en évitant la formation d'hernie sur cette membrane.

A cet effet, l'invention a pour objet un procédé de fabrication d'un réservoir contenant une substance active et adapté pour être introduit dans une cavité naturelle d'un être vivant, ce réservoir comprenant une membrane perméable à la substance active, caractérisé en ce qu'il comprend des étapes consistant à :
- a) placer au moins un tube destiné à constituer cette membrane dans au moins un organe de maintien, les extrémités du tube étant ouvertes,
- b) injecter par une des extrémités du tube, en évacuant tout air résiduel présent dans ce tube, une quantité d'un produit contenant une substance active sous forme pâteuse, cette quantité correspondant sensiblement au volume interne libre du tube,

- c) fermer, lorsque le tube est plein, l'extrémité du tube opposée à l'extrémité à partir de laquelle l'injection est réalisée,
- d) poursuivre l'injection du produit sous forme pâteuse dans le volume libre du tube jusqu'à obtention du diamètre externe désiré pour le réservoir en laissant le tube s'expandre radialement,
- e) effectuer la polymérisation du produit lorsque le réservoir a le diamètre externe désiré en maintenant le réservoir dans l'organe de maintien.

Un tel procédé permet, en une seule opération, de réaliser le réservoir et de le remplir de substance active. Il n'est plus nécessaire de fabriquer à part un cylindre de produit. L'injection du produit en absence d'air, sous pression, permet une répartition homogène de celui-ci dans le tube tout en évitant la présence d'hernies ou de bulles d'air.

Selon des aspects avantageux, mais non obligatoires de l'invention, le procédé peut incorporer une ou plusieurs des caractéristiques suivantes :
- Après l'étape e), on tronçonne le réservoir à la longueur finale désirée.
- Lors de l'étape a), on insère une tige dans le volume interne du tube.
- Lors de l'étape a), on place simultanément plusieurs tubes dans au moins un organe de maintien.
- On effectue les étapes b) à e) simultanément sur tous les tubes placés dans au moins un organe de maintien.

L'invention concerne également une installation permettant de mettre en oeuvre un procédé tel que décrit ci-dessus et, plus spécifiquement, un installation qui comprend :
- Au moins un organe de maintien d'au moins un tube destiné à former la membrane,
- au moins un organe d'injection d'un produit contenant la substance active dans le tube,
- au moins un organe de fermeture d'au moins une extrémité du tube après son remplissage avec le produit.
- Avantageusement, l'organe de maintien comporte au moins un moule définissant un logement de réception du tube lors de son remplissage et lors de la polymérisation du produit.
- L'organe de fermeture comprend une tige et un plateau d'obturation d'une extrémité du tube et d'une extrémité du logement.
- L'organe de maintien est équipé, sur une face interne, de points d'accrochage de la paroi du tube adaptés pour éviter le retrait et/ou l'extension longitudinale de la paroi lors de l'injection du produit et/ou de la polymérisation.
- La tige est recouverte d'un matériau non adhérent à la tige, notamment une gaine.
- Le logement comprend au moins une zone de réception d'au moins un organe de positionnement des extrémités du tube.
- Le logement comprend deux zones de réception, chacune étant disposée au voisinage d'une extrémité du logement et adaptée pour recevoir un organe de positionnement.
- Ces organes sont des bagues munies d'une collerette radiale.
- Ces bagues ont un diamètre interne voisin du diamètre externe désiré pour le réservoir.

L'invention sera mieux comprise et d'autres avantages de celle-ci apparaîtront plus clairement à la lecture de la description qui va suivre d'un procédé et de deux modes de réalisation d'une installation conformes à l'invention, donnée uniquement à titre d'exemple et faite en se référant aux dessins annexés dans lesquels :
- La figure 1 est une vue en perspective d'un stérilet hormonal équipé d'un réservoir réalisé selon l'invention,
- la figure 2 est une vue en perspective du réservoir du stérilet de la figure 1,
- la figure 3 est une vue en perspective, à la même échelle que la figure 2, d'un tube de silicone vide destiné à former la membrane entourant le réservoir de la figure 2,
- la figure 4 est une coupe schématique et longitudinale, à une autre échelle, d'un premier type de moule formant un moyen de maintien d'un tube de silicone lors de la mise en oeuvre du procédé,
- la figure 5 est une coupe schématique, longitudinale, à la même échelle que la figure 4, d'un premier moyen d'injection du produit dans le tube,
- les figures 6 à 10 sont des coupes schématiques, longitudinales, illustrant les différentes étapes du procédé utilisant les matériels des figures 4 et 5 et
- la figure 11 est une coupe schématique, latérale, longitudinale d'un second type de moule et d'un moyen d'injection du produit dans le tube.

Un stérilet intra-utérin 1 comprend un support 2 réalisé en un matériau inerte et non allergique à base de polymère. Ce support 2 a globalement la forme d'un T aux branches 3 recourbées en direction de sa tige 4, de manière à être configuré globalement en forme d'ancre de marine. Un réservoir 5 est positionné sur la tige 4 du support 2. Le réservoir est configuré en cylindre à base circulaire et pourvu d'une lumière centrale et longitudinale 6. Le diamètre D₆ de cette lumière est adapté pour permettre l'insertion à jeu réduit de la tige 4 dans le réservoir 5. Le réservoir 5 est bloqué en translation sur la tige 4 par deux bossages 70, 71 situés respectivement au voisinage des extrémités de la tige 4. Le bossage terminal 70 de la tige 4 est muni d'un orifice 72 permettant le passage de fils facilitant le retrait du stérilet dans la cavité utérine.

Le cylindre 5 comprend, en section transversale, outre la lumière centrale 6, une zone intermédiaire entre la paroi externe du réservoir et la lumière 6. Cette zone, dont l'épaisseur est généralement comprise entre 1 mm et 2 mm s'étend sur toute la longueur du réservoir 5. Elle est remplie par un tube 8 de produit solide. Ce produit solide comprend environ 20% à 40% d'un principe actif, notamment une hormone progestative, par exemple du levonorgestrel. La quantité d'hormone est suffisante pour assurer l'efficacité du dispositif pendant 2 à 5 ans avec une libération hormonale entre 20 et 25µg/24 heures ce qui permet une action contraceptive efficace. Le reste du produit 8 est composé essentiellement de silicone.

La paroi externe du réservoir 5 est formée par une membrane continue 9, réalisant ainsi un manchon protecteur autour du tube 8. Seules les sections terminales du cylindre 5 sont dépourvues de membrane. Cette membrane est à base d'un silicone ou d'un mélange de silicones. Ce ou ces silicones, sont avantageusement, de type différent de celui entrant dans la composition du produit 8, notamment en ce qui concerne la charge en silice.

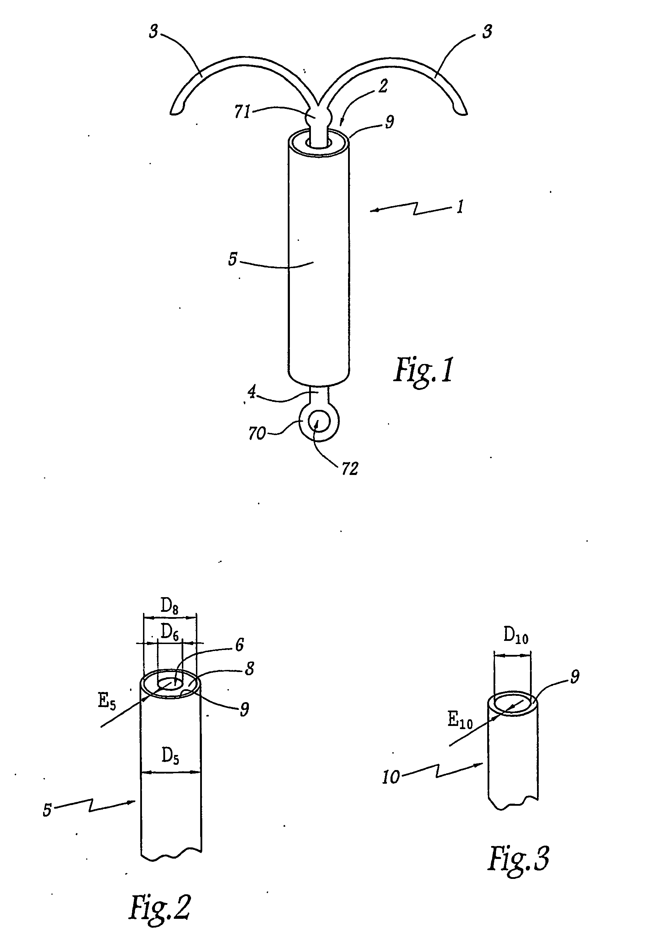

Comme illustré aux figures 2 et 3, la membrane tubulaire est formée à partir d'un tube 10 qui présente un diamètre interne D₁₀ environ 2 à 3 fois plus petit que le diamètre externe D₈ du produit 8. L'épaisseur E₁₀ de sa paroi est plus importante que l'épaisseur E₅ de la membrane 9 finale. L'épaisseur de la paroi est inversement proportionnelle au diamètre externe du tube.

L'extension radiale du tube 10 lui permet d'entourer le produit 8 en formant le réservoir 5 de diamètre externe D₅, avec une épaisseur E₅ de paroi suffisamment faible pour former une membrane. Les propriétés élastiques des silicones et leur porosité permettent de satisfaire les contraintes techniques évoquées précédemment. D'autres matériaux inertes, non allergiques, élastiques, extensibles et perméables aux substances actives sont utilisables pour la réalisation d'un réservoir 5 par un procédé tel que décrit ci-après.

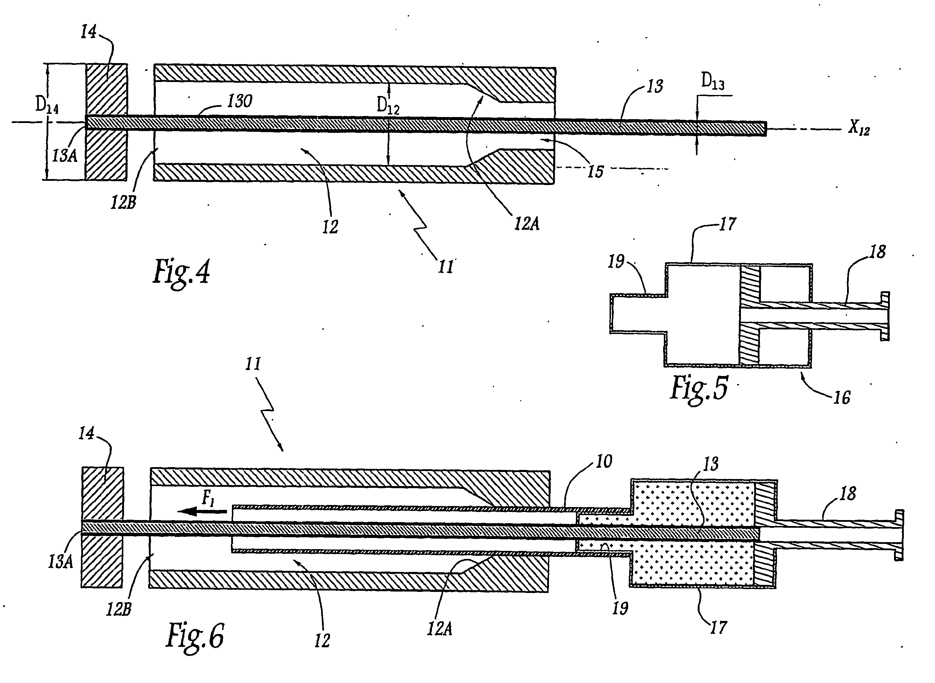

Comme représenté à la figure 4 un premier type de moule, en deux parties séparables, est réalisé dans un matériau rigide mais poreux, de manière à permettre le passage d'air humide entre l'intérieur et l'extérieur du moule. En effet, la polymérisation de certains silicones se fait en présence d'eau, dans ce cas il est nécessaire de maintenir une hygrométrie élevée à l'intérieur du moule. Avantageusement, le moule est en aluminium, ou en un alliage d'aluminium, et pourvu de petits orifices, par exemple d'un diamètre compris entre 0,2 et 0,3 mm, régulièrement répartis sur le moule. En variante, le moule peut être en un matériau fritté ou en silice comprimé. La partie principale 11 du moule définit un logement central 12 cylindrique à base circulaire et centré sur un axe X₁₂. Le diamètre interne D₁₂ du logement 12 est sensiblement identique au diamètre externe final D₅ du réservoir 5.

Une tige 13, par exemple métallique, est positionnée longitudinalement dans le logement 12, selon l'axe X₁₂. Cette tige a un diamètre externe D₁₃ correspondant sensiblement au diamètre interne du réservoir 5 fini, c'est-à-dire au diamètre D₆ de la lumière 6. La tige 13 est solidaire, au niveau de l'une de ses extrémités 13A, d'un support ou plateau 14, globalement en forme de disque, de diamètre D₁₄ supérieur au diamètre D₁₂. La tige 13 traverse, sur toute sa longueur, le moule 11, sans toucher les parois du logement 12. A une extrémité, le logement 12 communique à travers un convergent 12A avec un orifice 15 de diamètre inférieur au diamètre D₁₂.

Le plateau 14 est disposé à l'extérieur du moule 11, du côté du débouché 12B du logement 12 opposé au convergent 12A.

L'orifice 15 permet l'insertion d'un embout d'un premier type de moyen d'injection représenté schématiquement à la figure 5. Ce moyen d'injection 16, globalement en forme de seringue, comprend un corps principal 17 dans lequel un piston 18 se déplace de manière étanche. Ce piston 18 est creux, de manière à permettre le passage de la tige 13 lorsque l'embout d'insertion 19 du corps 17 est en place dans l'orifice 15 du moule 11. Ce dispositif d'injection 16 est relié, éventuellement, à une réserve de produit de manière à pourvoir fonctionner en continu.

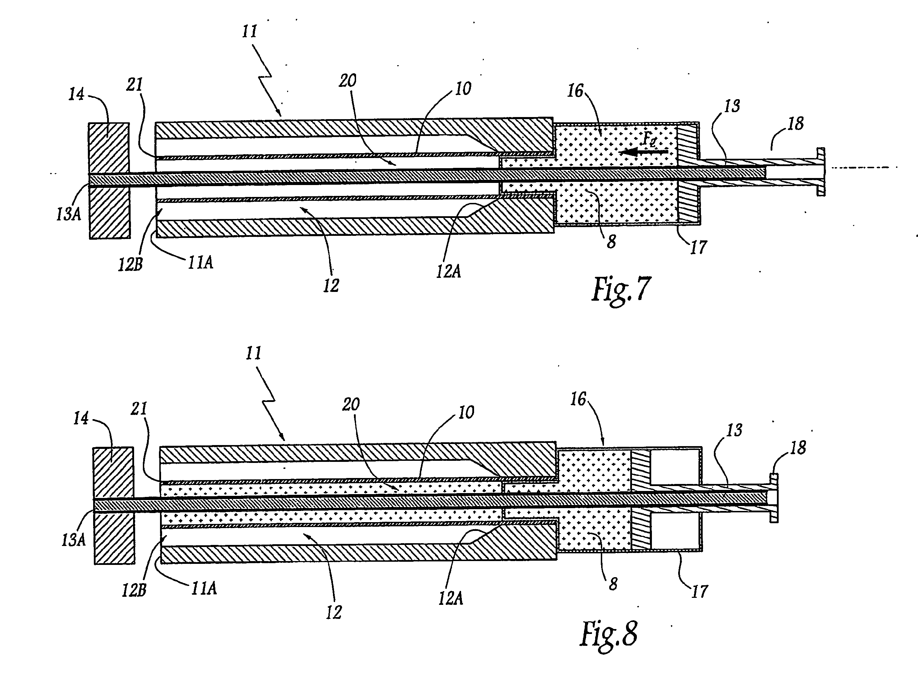

Comme représenté à la figure 6, la première étape du procédé de fabrication du réservoir 5 consiste à positionner, dans le sens de la flèche F₁ à la figure 6 et sur la tige 13, le tube ou manchon 10 de silicone après avoir inséré l'embout 19, en force, dans une extrémité du tube 10. Une fois que le manchon 10 coiffe la tige 13, l'embout 19 du système d'injection 16 est positionné dans le moule comme représenté à la figure 7.

Un mouvement du piston 18 en direction de l'autre extrémité 11A du moule, selon la flèche F₂, déplace vers l'espace annulaire 20 compris entre la tige 13 et la face interne du tube 10 le produit 8 sous forme pâteuse. Ce produit 8, qui comporte le principe actif, présente une viscosité qui varie en fonction du pourcentage de principe actif. La viscosité d'un produit à base de silicones n'est pas mesurée directement. Il est connu, pour les silicones, d'apprécier indirectement cette viscosité par une mesure de la vitesse d'écoulement du produit sous une pression donnée. Une méthode utilisée est donnée par la norme américaine ASTM-033 où la vitesse d'écoulement est exprimée en grammes par minute. En l'espèce, le produit 8 a une vitesse d'écoulement supérieure à 2 grammes par minute et de préférence, comprise entre 2,8 et 3 grammes par minute. En variante l'injection est effectuée par une vis sans fin ou un système à membrane.

L'injection du produit 8 se fait de manière lente, régulière et continue par exemple sous l'action d'une force, électrique, pneumatique ou mécanique exercée sur le piston 18. La quantité injectée est déterminée de manière à remplir sensiblement le volume annulaire 20 disponible dans le tube 10 de silicone ainsi maintenu dans le logement 12. Cette injection s'effectue sans déformation radiale et/ou longitudinale notable du tube 10, compte tenu de la quantité injectée et de la vitesse d'injection. Par ailleurs, le moule assure le maintien en place du tube 10.

Comme représenté aux figures 7 et 8, l'extrémité 21 du tube 10, opposée à l'orifice 15 reste ouverte pendant toute la durée de l'injection du produit 8. Une fois le tube 10 rempli, le remplissage étant facilité par l'ouverture de l'extrémité 21, ce qui permet d'évacuer tout l'air résiduel du volume 20, le piston 18 n'est toujours pas en butée contre l'embout 19 inséré dans l'extrémité du moule équipée de l'orifice 15, compte tenu de la quantité de produit 8 présente initialement dans le dispositif 16 comme représenté à la figure 8.

Lors de cette étape, le tube 10 est entièrement rempli par le produit 8, à l'exception du volume occupé pour la tige 13. Le produit est uniformément reparti dans l'espace annulaire 20, il est notamment exempt de bulles d'air au voisinage de la paroi du tube 10.

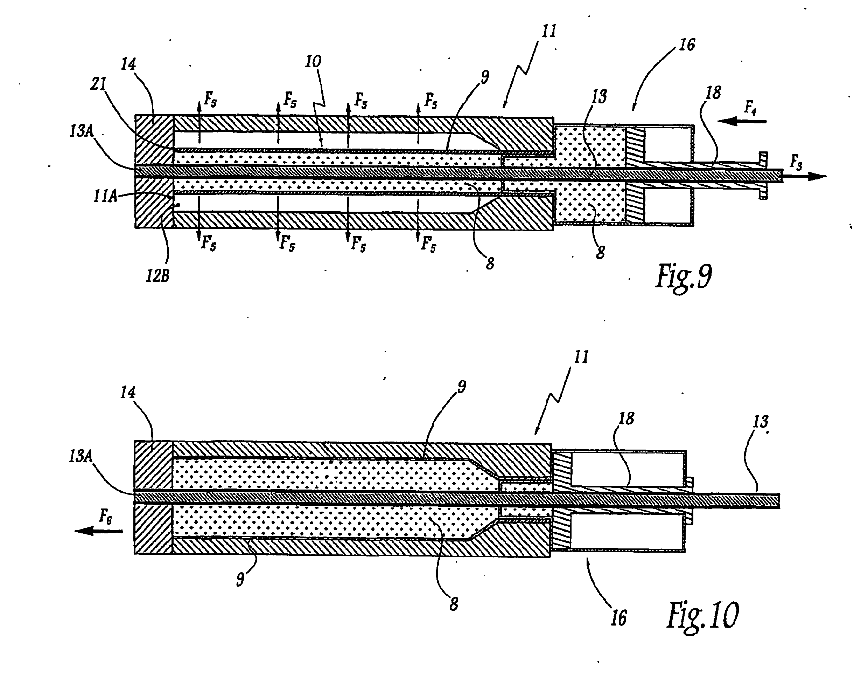

Dans l'étape suivante, illustrée à la figure 9, l'extrémité 21 du tube 10 est obturée par le plateau 14 du fait de son déplacement selon la flèche F₃ par coulissement sur la tige 13. Le plateau 14 ferme alors le débouché 12B et l'extrémité 21 du tube 10. Seul un passage pour la tige 13 est préservé dans le système de fermeture. La tige 13 est immobilisée par exemple, par un dispositif de mâchoires, de vannes à guillotine. En variante non représentée, l'extrémité 21 et le débouché 12B sont fermés par un système à guillotine ou à pince. Cette obturation de l'extrémité 21 étant effectuée, le produit 8 ne peut plus s'évacuer par cette extrémité du tube 10 opposée à la seringue 16. On poursuit l'injection du produit 8 dans le tube 10 selon la flèche F₄. Cette seconde injection a lieu sous une pression plus forte que la première. Compte tenu des propriétés élastiques de la paroi 9 du tube 10 en silicone, celle-ci se dilate radialement dans le sens des flèches F₅ et F'₅ jusqu'à venir au contact de la face interne 11A du moule 11 qui définit le logement 12. Du fait que la pression exercée par le produit 8 sur la paroi 9 est constante et uniformément répartie sur cette paroi 9, on dilate le tube 10 en évitant toute hernie et en réalisant une paroi 9 dont l'épaisseur finale est régulière en tout point de la paroi. On forme ainsi une membrane dont la porosité et le coefficient de diffusion sont optimaux et réguliers. La quantité de produit 8 injectée lors de cette étape est notamment fonction du diamètre final D₅ du réservoir 5, c'est-à-dire, en pratique, fonction du diamètre interne D₁₂ du logement 12.

L'injection est terminée lorsque la face externe de la paroi 9 du tube 10 et la face interne 11A du moule 11 sont en contact, sur toute leur surface respective.

Il convient alors d'attendre la polymérisation du produit dans le tube 10. La polymérisation d'un produit à base de silicone sur un élément lui-même en silicone permet une liaison efficace entre les composants, de manière analogue à un soudage. La porosité, à la fois de la paroi 9 et du matériau constitutif du moule 11, permet de maintenir dans le moule une hygrométrie suffisante pour assurer une polymérisation rapide et complète du produit 8. On réalise ainsi un réservoir 5 dans lequel le produit 8 est maintenu efficacement et régulièrement réparti dans la membrane 9, ce qui améliore la diffusion hormonale.

Afin d'éviter tout retrait et/ou extension longitudinale lors de l'injection du produit 8, la face interne 11A n'est pas lisse mais présente des aspérités, non représentées, suffisamment importantes pour former des points d'accrochage de la paroi 9 du tube 10 évitant ainsi son retrait et/ou son extension longitudinale lors de l'injection du produit 8 et/ou de la polymérisation. Si nécessaire, une pause dans l'injection est effectuée pour laisser le temps au tube 10 de reprendre ses dimensions longitudinales initiales.

On réalise ainsi, en une seule opération, la fabrication du produit 8 et son insertion dans le réservoir 5.

Dans une dernière étape, non illustrée, on retire la tige 13 du moule 11 dans le sens de la flèche F₆ à la figure 10, ce qui permet d'extraire le réservoir 5 du logement 12. La tige peut ensuite être extraite du réservoir.

Lors de la polymérisation, une adhérence du produit 8 sur la tige 13 peut compromettre un retrait aisé de cette dernière hors du moule 11.

Pour éviter cette adhérence, on recouvre la tige 13 d'un matériau non adhérent à la tige 13. Avantageusement il s'agit d'une gaine 130. Cette gaine 130, de faible épaisseur est réalisée dans un matériau biocompatible et inerte vis-à-vis des autres composants du stérilet 1.

Cette gaine 130 est positionnée sur la tige 13 préalablement à la mise en place de celle-ci dans le moule 11.

Outre un retrait aisé de la tige 13, cette gaine 130 participe à l'étanchéité entre les extrémités de la tige 13 avec le piston 18 et le plateau 14.

En variante, la gaine 130 est remplacée par un revêtement de surface de la tige 13 non adhérent au produit 8. Dans un autre mode de réalisation, le matériau constitutif de la tige 13 est lui-même non adhérent au produit 8.

Selon un aspect non représenté de l'invention, le moule 11 peut être formé de deux demi-coquilles complémentaires et définissant ensemble le logement 12. Dans ce cas, après la polymérisation, le moule est ouvert pour permettre le retrait du réservoir 5.

Lorsque la tige 13 a été retirée, l'ensemble précité est tronçonné à la longueur souhaitée en fonction de la longueur de la tige 4 qui est ensuite introduite dans la lumière 6. En pratique, la longueur du logement 12 peut permettre de réaliser plusieurs réservoirs 5 bout à bout.

Dans une variante non représentée, le moule 11 comporte plusieurs gorges 12 disposées en parallèle et/ou en étoile, permettant ainsi la réalisation simultanée et en parallèle, de plusieurs réservoirs. Le dispositif d'injection 16 est adapté en conséquence.

En variante, le dispositif d'injection 16 est équipé d'un moyen d'alimentation en continu du corps principal 17 avec du produit 8.

Dans un autre mode de réalisation le moule 11 est dépourvu de tige analogue à la tige 13. Le réservoir obtenu est un cylindre plein. Il est alors nécessaire de réaliser un support différent de celui décrit précédemment. Ce peut être, par exemple, un support équipé de moyens d'accrochage de type anneau ouvert.

En variante, l'installation comprend un moule 11 dont la longueur du logement 12 est plus faible que celle décrite. Ces moules sont adaptés pour recevoir, en lieu et place de la tige centrale 13, la tige 4 d'un support 2. Dans ce cas, on réalise en une seule opération le stérilet hormonal prêt à l'usage par surmoulage du réservoir 5 sur la tige 4. Dans ce cas, la longueur du logement 12 correspond sensiblement à la longueur du stérilet 1, l'étape de tronçonnage n'étant plus nécessaire.

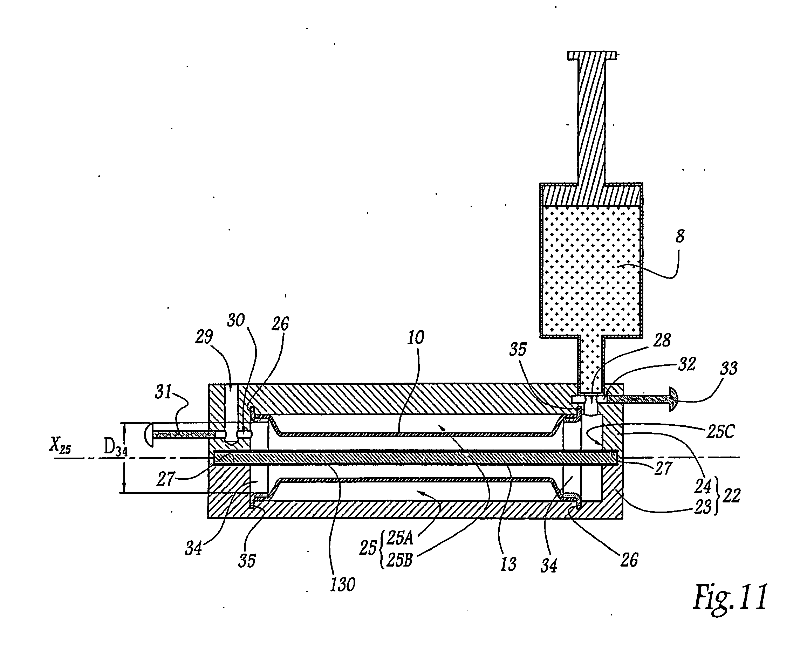

La figure 11 illustre un autre type de moule et un autre moyen d'injection du produit conforme à l'invention. Cet autre moule 22 comprend un demi moule inférieur 23 et un demi moule supérieur 24. Le moule 22 est réalisé dans un matériau similaire à celui du moule 11 afin de permettre le maintien d'une hygrométrie élevée dans le moule.

Le demi moule inférieur 23 comprend un logement central 25A de dimensions et de forme adaptées aux dimensions souhaitées du réservoir 5. En l'espèce, le logement 25A est, semi-cylindrique à base circulaire. Au voisinage des extrémités de ce logement 25A, des entailles 26 sont ménagées.

Les sections d'extrémité du demi moule 23 sont équipées, en partie supérieure, d'une rainure 27. Ces rainures 27 ont une forme et des dimensions adaptées pour recevoir la tige 13.

Le demi moule supérieur 24 est similaire au demi moule 23. Il comprend un logement central 25B semi-cylindrique à base circulaire. Ce logement 25B, similaire au logement 25A, a des dimensions et une forme adaptée aux dimensions souhaitées du réservoir 5. En particulier, le demi moule 24 est équipé de rainures 27 et d'entailles 26 de formes et de dimensions similaires à celles du demi moule 23. Les entailles 26 et les rainures 27 de chaque demi moule 23, 24 sont avantageusement placées en regard lorsque le moule 22 est fermé. Dans cette configuration, le moule 22 comprend un logement central 25 formé par les logements 25A et 25B des demi-moules 23 et 24.

Le demi moule 24 est pourvu de deux orifices 28, 29 disposés au voisinage de ses extrémités. Ces orifices 28, 29 sont perpendiculaires à un axe longitudinal X₂₅ du logement 25B lorsque le moule est fermé. Ils permettent une communication entre l'extérieur du moule 22 et le logement 25 lorsque le moule est fermé.

L'orifice 28 est traversant et débouche dans le logement 25B, entre une entaille 26 et la paroi 25C d'extrémité du logement 25B. Le débouché extérieur de cet orifice a un diamètre interne voisin du diamètre externe de l'embout 19 d'une seringue 16.

L'orifice 29 ménagé sur l'autre extrémité du demi-moule 24 forme un puits borgne. Cet orifice est traversé, au voisinage de son extrémité fermée, par un canal 30. Ce canal est orienté selon une direction globalement parallèle à l'axe X₂₅ lorsque le moule est fermé. Le canal 30 met en communication l'extérieur et le logement 25. Ce canal 30 reçoit un organe obturateur 31, par exemple un pion en polymère rigide. Ce pion 31 est déplaçable dans le canal 30, de manière à obturer ce dernier et empêcher toute communication, par le canal 30 ou l'orifice 29, entre le logement 25 et l'extérieur.

L'orifice 28 est également traversé par un canal 32 dans lequel se déplace un organe obturateur 33 en forme de pion. Ce canal 32 est borgne, son extrémité fermée étant située dans la paroi du demi moule 24. Il est disposé au voisinage du débouché de l'orifice 28. Le canal 32 est orienté parallèlement à l'axe X₂₅ lorsque le moule est fermé.

Ces organes d'obturation 31, 33 ont une longueur et un diamètre suffisants pour obturer de manière étanche les canaux 30, 32 correspondants.

Lorsque l'on veut réaliser un stérilet 1 on insère à chaque extrémité d'un tube 10, par exemple au moyen d'une pince de type écarteur, un organe de positionnement 34. Cet organe est formé par une bague 34 réalisée en un matériau rigide, inerte et biocompatible. Cette bague 34 est équipée d'une collerette radiale 35 s'étendant vers l'extérieur. Le diamètre interne D₃₄ de la bague 34 est voisin du diamètre externe D₅ souhaité du réservoir 5. Ainsi les extrémités du tube 10 coiffant les bagues 34 ont un diamètre proche de leur diamètre final lorsque le réservoir 5 est réalisé. La tige 13, également recouverte d'une gaine 130 analogue à celle mentionnée ci-dessus, est ensuite insérée dans la lumière du tube 10. L'ensemble est alors positionné dans le demi moule inférieur 23, de manière à ce que les extrémités de la tige 13 reposent dans les rainures 27. On positionne le tube 10 dans le demi moule 23 de manière à ce que les collerettes 35 des bagues 34 d'extrémité soient insérées, à jeu réduit dans les entailles 26 correspondantes.

On referme le moule 22 en rabattant le demi moule supérieur 24. Ainsi, les rainures 27 et les entailles 26 du demi moule 24 coiffent les parties libres de la tige 13 et des collerettes 35. On maintient et on positionne ainsi exactement le tube 10 et la tige 13 dans le moule 22.

Dans une première étape, similaire à celle précédemment décrite, on injecte le produit 8 sous forme pâteuse par l'orifice 28 après avoir inséré l'embout 19 d'une seringue dans le débouché de ce dernier. Le pion 33 est en position de retrait afin de permettre le passage du produit dans les logements 25A et 25B. Le produit 8 pénètre dans le tube 10. L'air contenu dans le tube 10 est repoussé hors du moule 22 et sort par l'orifice 29 dont le passage est libre, le pion 31 étant en position de retrait.

Lorsque le produit pâteux 8 occupe l'ensemble de l'espace disponible entre le tube 10 et la gaine 130 concentriques, on obture l'orifice 29 en repoussant le pion 31 en direction du logement 25B. On poursuit alors l'injection du produit 8 jusqu'à obtenir le diamètre désiré du réservoir 5. lors de l'injection, le tube 10 est maintenu en place par les bagues 34, il ne peut donc y avoir d'expansion longitudinale du tube 10, seule l'expansion radiale est permise. En fin d'opération on obture l'orifice 28 d'injection en repoussant le pion 33 au fond du canal 32.

La polymérisation est effectuée en milieu humide de manière similaire à celle précédemment décrite. En fin de procédé, on ouvre le moule et l'on retire aisément la tige 13 qui coulisse librement à l'intérieur de la gaine 130. On tronçonne le réservoir à la longueur voulue. Cette découpe s'effectue notamment au niveau des bagues 34.

En variante, on dispose plusieurs moules 22 en parallèles, alimentés par un dispositif de type seringue à embouts multiples de manière à réaliser, en parallèles, plusieurs réservoirs 5.

## Revendications

1. Procédé de fabrication d'un réservoir (5) contenant une substance active et adapté pour être introduit dans une cavité naturelle d'un être vivant, ledit réservoir (5) comprenant une membrane (9) perméable à ladite substance active, le procédé comprenant des étapes consistant à:
- a) placer au moins un tube (10) destiné à constituer ladite membrane dans au moins un organe de maintien (11; 22), les extrémités dudit tube étant ouvertes,
- b) injecter (16) par une desdites extrémités dudit tube (10), en évacuant tout air résiduel présent dans ledit tube, une quantité d'un produit (8) contenant une substance active sous forme pâteuse, cette quantité correspondant sensiblement au volume interne libre (20) dudit tube (10),
- c) fermer, lorsque ledit tube (10) est plein, l'extrémité (21) du tube opposée a l'extrémité à partir de laquelle l'injection (16) est réalisée,
- d) poursuivre l'injection (16) dudit produit (8) sous forme pâteuse dans ledit volume libre (20) jusqu'à obtention du diamètre (D₅) externe désiré pour ledit réservoir (5) en laissant ledit tube s'expandre radialement (F₅, F'₅),
- e) effectuer la polymérisation dudit produit (8) lorsque le réservoir (5) a le diamètre (D₅) externe désiré en maintenant le réservoir (5) dans l'organe de maintien (11; 22).

2. Procédé selon la revendication 1, **caractérisé en ce qu'**après l'étape e), on tronçonne le réservoir (5) à la longueur finale desirée.

3. Procédé selon l'une des revendications précédentes, **caractérisé en ce que** lors de l'étape a), on insère une tige (13) dans le volume interne (20) dudit tube (10).

4. Procédé selon l'une des revendications précédentes, **caractérisé en ce que** lors de l'étape a), on place simultanément plusieurs tubes (10) dans au moins un organe de maintien.

5. Procédé selon la revendication 4, **caractérisé en ce qu'**on effectue les étapes b) à e) simultanément sur tous les tubes (10) placés dans au moins un organe de maintien.

6. Installation pour réaliser un réservoir (5) contenant une substance active et adapté pour être introduit dans une cavité naturelle d'un être vivant, ledit réservoir étant enveloppé par une membrane (9) perméable à ladite substance active, comprenant
- au moins un organe de maintien (11; 22) d'au moins un tube (10) destiné à former ladite membrane (9),
- au moins un organe d'injection (16, 17, 18) d'un produit (8) contenant la substance active dans ledit tube,
- au moins un organe de fermeture (14, 13; 31) d'au moins une extrémité (21) dudit tube après son remplissage avec le produit,
**caractérisée en ce que** ledit organe de maintien comporte au moins un moule (11; 22) définissant un logement (12; 25) de réception dudit tube (10) lors de son remplissage et lors de la polymérisation dudit produit (8).

7. Installation selon la revendication 6, **caractérisée en ce que** l'organe de fermeture comprend une tige (13) et un plateau (14) d'obturation d'une extrémité (21) du tube (10) et d'une extrémité (12B) du logement (12).

8. Installation selon la revendication 6, **caractérisée en ce que** ledit organe de maintien (11) est équipé, sur une face interne (11A), de points d'accrochage de la paroi (9) du tube (10) adaptés pour éviter le retrait et/ou l'extension longitudinale de ladite paroi (9) lors de l'injection du produit (8) et/ou de la polymérisation.

9. Installation selon la revendication 7, **caractérisée en ce que** la tige (13) est recouverte d'un matériau non adhérent à la tige (13), notamment une gaine (130).

10. Installation selon la revendication 6, **caractérisée en ce que** ledit logement (25) comprend au moins une zone (26) de réception d'au moins un organe de positionnement (34, 35) des extrémités du tube (10).

11. Installation selon la revendication 10, **caractérisée en ce que** le logement (25) comprend deux zones de réception (26), chacune étant disposée au voisinage d'une extrémité du logement (25) et adaptée pour recevoir un organe de positionnement (34, 35).

12. Installation selon la revendication 11, **caractérisée en ce que** lesdits organes sont des bagues (34) munies d'une collerette radiale (35).

13. Installation selon la revendication 12, **caractérisée en ce que** lesdites bagues (34) ont un diamètre interne (D₃₄) voisin du diamètre externe (D₅) désiré pour le réservoir (5).

## Claims

1. A method of making a reservoir (5) containing an active substance and suitable for being inserted into a natural cavity of a living being, said reservoir (5) comprising a membrane (9) that is permeable to said active substance, the method comprising steps consisting in:
- a) placing at least one tube (10) designed to form said membrane in at least one retention member (11; 22), the ends of said tube being open,
- b) injecting (16) through one of said ends of said tube (10), while expelling all the residual air present in said tube, a quantity of a product (8) containing an active substance in paste form, this quantity corresponding substantially to the internal free volume (20) of said tube (10),
- c) when said tube (10) is full, closing the end (24) of the tube opposite to the end from which the injection (16) is performed,
- d) continuing the injection (16) of said product (8) in paste form into said free volume (20) until the desired external diameter (D₅) for said reservoir (5) is obtained by allowing said tube to expand radially (F₅, F'₅,)
- e) polymerizing said product (8) when the reservoir (5) has the desired external diameter (D₅) while keeping the reservoir (5) in the retention member (11; 22).

2. The method according to claim 1, **characterized in that**, after step e), the reservoir (5) is cut to the desired final length.

3. The method according to one of the preceding claims, **characterized in that**, during step a), a rod (13) is inserted into the internal volume (20) of said tube (10).

4. The method according to one of the preceding claims, **characterized in that**, during step a), several tubes (10) are simultaneously placed in at least one retention member.

5. The method according to claim 4, **characterized in that** steps b) to e) are carried out simultaneously on all the tubes (10) placed in a t least one retention member.

6. An installation for making a reservoir (5) containing an active substance and suitable for being inserted into a natural cavity of a living being, said reservoir being enveloped by a membrane (9) permeable to said active substance, comprising
- at least one member (11; 22) for the retention of at least one tube (10) designed to form said membrane (9),
- at least one member (16, 17, 18) for the injection of a product (8) containing the active substance into said tube,
- at least one member (14, 13; 31) for the closure of at least one end (21) of said tube after it has been filled with the product,
**characterized in that** the retention member comprises at least one mold (11; 22) defining a housing (12; 25) for receiving said tube (10) when it is filled and when said product (8) is polymerized.

7. The installation according to claim 6, **characterized in that** the closure member comprises a rod (13) and a plate (14) for blanking off one end (21) of the tube (10) and one end (12B) of the housing (12).

8. The installation according to claim 6, **characterized in that** said retention member (11) is fitted, on an internal face (11A), with coupling points of the wall (9) of the tube (10) suitable for preventing the retraction and/or the longitudinal extension of said wall (9) when the product (8) is injected and/or polymerized.

9. The installation according to claim 7, **characterized in that** the rod (13) is covered with a material not adhering to the rod (13), particularly a sheath (130).

10. The installation according to claim 6, **characterized in that** said housing (25) comprises at least one zone (26) for receiving at least one member (34, 35) for positioning the ends of the tube (10).

11. The installation according to claim 10, **characterized in that** the housing (25) comprises two reception zones (26), each being placed in the vicinity of an end of the housing (25) and suitable for receiving a positioning member (34, 35).

12. The installation according to claim 11, **characterized in that** said members are rings (34) furnished with a radial collar (35).

13. The installation according to claim 12, **characterized in that** said rings (34) have an internal diameter (D₃₄) close to the external diameter (D₅) desired for the reservoir (5).

## Patentansprüche

1. Verfahren zur Herstellung eines Behälters (5), der eine aktive Substanz enthält und dazu geeignet ist, in einen natürlichen Hohlraum eines Lebewesens eingeführt zu werden, wobei der Behälter (5) eine für die aktive Substanz durchlässige Membran (9) umfasst, wobei das Verfahren die folgenden Schritte umfasst:
- a) Anordnen mindestens eines Rohres (10), das dazu bestimmt ist, die Membran zu bilden, in mindestens einem Halteelement (11; 22), wobei die Enden des Rohrs offen sind,
- b) Einspritzen (16) einer Menge eines Produkts (8), das eine aktive Substanz in Pastenform enthält, durch eines der Enden des Rohrs (10) unter Beseitigung der gesamten restlichen Luft, die im Rohr enthalten ist, wobei diese Menge im Wesentlichen dem freien Innenvolumen (20) des Rohrs (10) entspricht,
- c) Schließen jenes Endes (21) des Rohrs, das dem Ende, von dem aus das Einspritzen (16) erfolgte, gegenüberliegt, wenn das Rohr (10) voll ist,
- d) Fortsetzen des Einspritzens (16) des Produkts (8) in Pastenform in das freie Volumen (20), bis der gewünschte Außendurchmesser (D₅) für den Behälter (5) erreicht ist, wobei sich das Rohr radial ausdehnen kann (F₅, F'₅),
- e) Durchführen der Polymerisation des Produktes (8), wenn der Behälter (5) den gewünschten Außendurchmesser (D₅) aufweist, wobei der Behälter (5) in dem Halteelement (11; 22) gehalten wird.

2. Verfahren nach Anspruch 1, **dadurch gekennzeichnet, dass** nach Schritt e) der Behälter (5) auf die gewünschte endgültige Länge abgeschnitten wird.

3. Verfahren nach einem der vorhergehenden Ansprüche, **dadurch gekennzeichnet, dass** in Schritt a) ein Stab (13) in das Innenvolumen (20) des Rohrs (10) eingesetzt wird.

4. Verfahren nach einem der vorhergehenden Ansprüche, **dadurch gekennzeichnet, dass** in Schritt a) gleichzeitig mehrere Rohre (10) in mindestens ein Halteelement eingesetzt werden.

5. Verfahren nach Anspruch 4, **dadurch gekennzeichnet, dass** die Schritte b) bis e) gleichzeitig an allen Rohren (10), die in mindestens einem Halteelement angeordnet werden, durchgeführt werden.

6. Anlage zur Herstellung eines Behälters (5), der eine aktive Substanz enthält und dazu geeignet ist, in einen natürlichen Hohlraum eines Lebewesens eingeführt zu werden, wobei der Behälter von einer für die aktive Substanz durchlässigen Membran (9) umgeben ist, umfassend:
- mindestens ein Halteelement (11; 22) für mindestens ein Rohr (10), das dazu bestimmt ist, die Membran (9) zu bilden,
- mindestens ein Einspritzelement (16, 17, 18) für ein die aktive Substanz enthaltendes Produkt (8) in das Rohr,
- mindestens ein Verschlusselement (14, 13; 31) für mindestens ein Ende (21) des Rohrs nach seiner Befüllung mit dem Produkt,
**dadurch gekennzeichnet, dass** das Halteelement mindestens eine Form (11; 22) umfasst, die eine Aufnahmelagerung (12; 25) für das Rohr (10) bei seiner Befüllung und bei der Polymerisation des Produkts (8) umfasst.

7. Anlage nach Anspruch 6, **dadurch gekennzeichnet, dass** das Verschlusselement einen Stab (13) und eine Platte (14) für den Verschluss eines Endes (21) des Rohrs (10) und eines Endes (12B) der Lagerung (12) umfasst.

8. Anlage nach Anspruch 6, **dadurch gekennzeichnet, dass** das Halteelement (11) auf einer Innenseite (11A) mit Verankerungspunkten für die Wand (9) des Rohrs (10) versehen ist, die dazu geeignet sind, die Schrumpfung und/oder die Längsausdehnung der Wand (9) beim Einspritzen des Produkts (8) und/oder bei der Polymerisation zu vermeiden.

9. Anlage nach Anspruch 7, **dadurch gekennzeichnet, dass** der Stab (13) mit einem nicht an dem Stab (13) anhaftenden Material, insbesondere einem Mantel (130), überzogen ist.

10. Anlage nach Anspruch 6, **dadurch gekennzeichnet, dass** die Lagerung (25) mindestens eine Aufnahmezone (26) für mindestens ein Element (34, 35) zum Positionieren der Enden des Rohrs (10) umfasst.

11. Anlage nach Anspruch 10, **dadurch gekennzeichnet, dass** die Lagerung (25) zwei Aufnahmezonen (26) umfasst, die jeweils in der Nähe eines Endes der Lagerung (25) angeordnet und dazu geeignet sind, ein Positionierelement (34, 35) aufzunehmen.

12. Anlage nach Anspruch 11, **dadurch gekennzeichnet, dass** die Elemente Ringe (34) sind, die mit einem radialen Kragen (35) versehen sind.

13. Anlage nach Anspruch 12, **dadurch gekennzeichnet, dass** die Ringe (34) einen Innendurchmesser (D₃₄) ähnlich dem für den Behälter (5) gewünschten Außendurchmesser (D₅) aufweisen.
